**Europäisches Patentamt**

**European Patent Office**

**Office européen des brevets**

(19)

(11) Veröffentlichungsnummer: **0 213 128**
**B1**

(12) ## EUROPÄISCHE PATENTSCHRIFT

(45) Veröffentlichungstag der Patentschrift: **11.04.90**

(51) Int. Cl.⁵: **A 61 M 5/14**

(21) Anmeldenummer: **85901906.9**

(22) Anmeldetag: **30.04.85**

(86) Internationale Anmeldenummer:
**PCT/AT85/00013**

(87) Internationale Veröffentlichungsnummer:
**WO 85/05041 21.11.85 Gazette 85/25**

(54) **DURCHFLUSSREGULATOR.**

(30) Priorität: **02.05.84 AT 1441/84**

(43) Veröffentlichungstag der Anmeldung:
**11.03.87 Patentblatt 87/11**

(45) Bekanntmachung des Hinweises auf die
Patenterteilung:
**11.04.90 Patentblatt 90/15**

(84) Benannte Vertragsstaaten:
**BE CH DE FR GB IT LI LU NL SE**

(56) Entgegenhaltungen:
**DE-A-2 735 955**
**DE-B-1 120 230**
**FR-A-2 227 019**
**FR-A-2 432 122**
**US-A-4 142 523**

(73) Patentinhaber: **NAGY, Erich**
**Dr. Natterergasse 6/2/97**
**A-1020 Wien (AT)**

(72) Erfinder: **NAGY, Erich**
**Dr. Natterergasse 6/2/97**
**A-1020 Wien (AT)**

(74) Vertreter: **Brauneiss, Leo**
**Patentanwälte Dipl.-Ing. Leo Brauneiss, Dipl.-**
**Ing. Dr. Helmut Wildhack Strohgasse 10**
**A-1030 Wien (AT)**

Courier Press, Leamington Spa, England.

**Beschreibung**

Die Erfindung betrifft einen Durchflußregulator für Schwerkraftinfusion bzw.- Transfusionen, mit einem Gehäuse, des eine Einlaßöffnung und eine am Gehäuseumfang um 180° versetzte Auslaßöffnung für die Flüssigkeit aufweist, und mit einem im Gehäuse angeordneten, relativ zum Gehäuse drehbaren, zylindrischen Ventilkörper, dessen Mantel eine Drosselnut mit sich allmählich veränderndem Querschnitt zur Steuerung des Durchflusses zwischen der Einlaßöffnung und der Auslaßöffnung aufweist, welche Drosselnut sich im Ventilkörper in einem achsnormalen Querschnitt über einen Teil des Umfanges, insbesondere über einen Winkelbereich von etwa 320°, des Ventilkörpers ersteckt und in einer Ebene liegt, in welcher die Achse der Auslaßöffnung des Gehäuses liegt, so daß, stromabwärts betrachtet, je nach Winkelstellung des Ventilkörpers ein größerer oder kleinerer Querschnittsbereich der Drosselnut mit der Auslaßöffnung im Verbindung steht und dadurch eine größere oder kleinere Anzahl von Tropfen pro Zeiteinheit aus dem Gehäuse abgegeben werden kann.

Einige bekannte Durchflußregulatoren (AT—B—358 160, DE—A—2 420 966, DE—B—2 147 572) sind ohne zylindrischen Ventilkörper, der mit Umfangsnuten versehen ist, ausgebildet und weisen eine komplizierte Konstrukton auf, die im Hinblick auf die Herstellungskosten ungünstig ist.

Bei einer bekannten Konstruktion der eingangs beschriebenen Art (DE—A—2 735 955) liegen die Einlaßöffnung, die Drosselbut und die Auslaßöffnung in einer gemeinsamen Ebene. Dies hat den Nachteil, daß eine auch nur geringfügige Verdrehung des Ventilkörpers zur Folge haben kann, daß der Durchflußregulator von der Abschlußstellung auf volle Durchlaßstellung verstellt wird. Ein weiterer Nachteil besteht darin, daß nur etwa der halbe Umfang des Ventilkörpers für eine Änderung des Durchflußquerschnittes wirksam zur Verfügung steht, so daß die Regelung verhältnismäßig grob ist.

Auf dem Gebiete der Regelung unter Druck stehender Flüssigkeiten, z.B. bei der Einspritzung von zur Verbrennung bestimmtem Kraftstoff, ist es bekannt (DE—B—1 120 230, FR—A—2 432 122), das zu regelnde Medium über eine Ringnut zuzuführen, die über eine zentrale Bohrung oder eine Längsnut mit einer Steuernut in Verbindung steht, deren Querschnitt sich in Umfangsrichtung des Steuerzylinders verändert. Diese Steuernut ist aber stets nur sehr kurz, so daß nur eine verhältnismäßig grobe Regelmöglichkeit besteht, abgesehen davon, daß sich Konstruktionen, die für unter Druck stehende Flüssigkeiten bestimmt sind, nicht ohne weiteres auf Durchflußregulatoren für Schwerkraftinfusionen bzw.- transfusionen übertragen lassen. Schließlich ist der Aufwand bei den bekannten Konstruktionen relativ hoch, was sich gerade bei zur Einmalverwendung bestimmten Durchflußregulatoren nachteilig auswirkt.

Es ist Ziel der Erfindung, einen Durchflußregulator der eingangs geschilderten Art so zu verbessern, daß er sich vorteilhaft von den bekannten Geräten unterscheidet, im Hinblick auf die Herstellungskosten sehr günstig liegt und bei Vorrichtungen besonders gut einsetzbar ist, die zum Zuführen von Flüssigkeiten in den Körper dienen, bei welchen der Durchfluß regulator zwischen zwei fluidführenden Schläuchen eingesetzt wird. Die Erfindung löst diese Aufgabe — ausgehend von einem Durchflußregulator der eingangs beschriebenen Art — dadurch, daß ein vom größten Querschnittsabschnitt der Drosselnut ausgehender, sich insbesondere in Achsrichtung des Ventilkörpers erstreckender Kanal in Form einer Mantelnut mit einer sich über 360° erstreckenden Umfangsnut des Ventilkörpers in Verbindung steht, in die der Einlaß in das Gehäuse mündet, welcher relativ zum Auslaß in Achsrichtung des Ventilkörpers versetzt ist, so daß, stromaufwärts betrachtet, die Drosselnut über den Kanal und die Umfangsnut mit der Einlaßöffnung in Verbindung steht. Diese Umfangsnut stellt im Zusammenhang mit dem mit ihr verbundenen Kanal stets eine der maximalen Durchflußrate angepaßte Flüssigkeitsmenge zur Verfügung, welche über die Drosselnut je nach der Winkelstellung des Ventilkörpers auf die jeweils eingestellte Durchflußrate gedrosselt wird, so daß ein größerer oder kleinerer Querschnittsbereich der Drosselnut mit der Auslaßöffnung in Verbindung steht und dadurch eine größere oder kleinere Anzahl von Tropfen pro Zeiteinheit aus dem Gehäuse abgegeben werden kann. Hiebei ist es möglich, die gesamte in Umfangsrichtung des Ventilkörpers gemessene Länge der Drosselnut wirksam auszunützen, so daß eine feine Regelungsmöglichkeit besteht. Weiters ist vorteilhaft, daß sich erfindungsgemäße Durchflußregulatoren einfach und billig in Kunststoff herstellen lassen.

Gemäß einer bevorzugten Ausführungsform der Erfindung ist der Ventilkörper außerhalb des Gehäuses mit einer Betätigungsscheibe versehen, an der erforderlichenfalls eine Skale angebracht werden kann, mit der die verschiedenen Durchflußraten angezeigt werden.

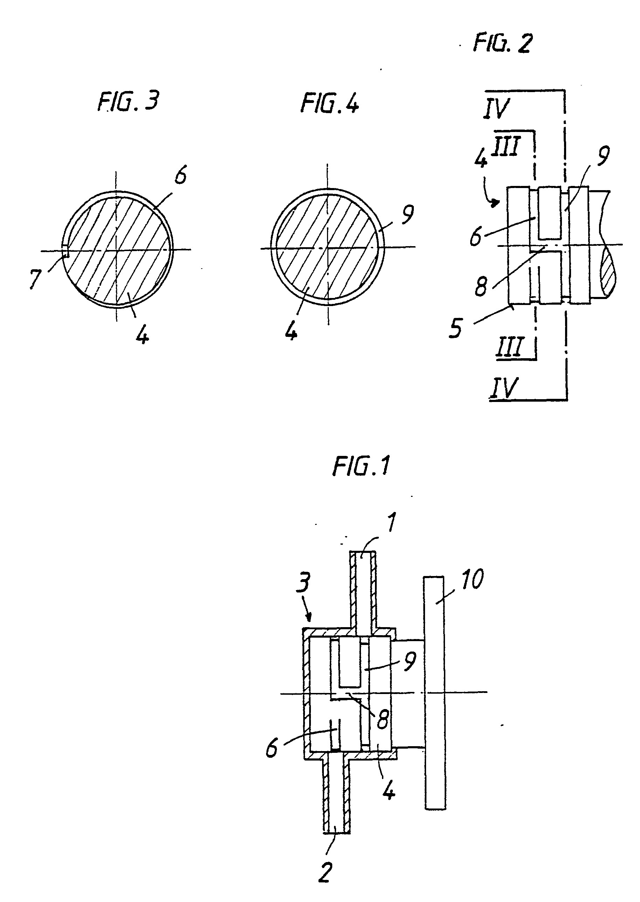

Anhand der Zeichnung wird eine Ausführungsform des Erfindungsgegenstandes im folgenden näher erläutert. Fig. 1 zeigt einen Längsschnitt durch den Durchflußregulator. Fig. 2 zeigt den Ventilkörper in seitenansicht. Die Fig. 3 und 4 sind Schnitte nach den Linien III—III bzw. IV—IV der Fig. 2.

Der dargestellte Durchflußregulator besteht aus nur zwei Elementen, nämlich aus einem Ventilkörper 4 mit Betätigungsscheibe 10 und aus einem Gehäuse 3, in welchem der Ventilkörper 4 verdreht werden kann. Der Ventilkörper 4 besitzt eine Umfangsnut 9, die in der Ebene liegt in der die Achse der Einlaßöffnung 1 des Gehäuses 3 liegt. Über einen Kanal 8 in Form einer Mantelnut ist die Umfangsnut 9 mit einer weiteren, sich im Querschnitt verjüngenden, am Umfang des Ventilkörpers 4 verlaufenden Nut 6 verbunden, welche eine Drosselnut bildet, sich in einem achsnormalen Querschnitt des Ventilkörpers erstreckt, und in der Ebene liegt, in der die Achse der Auslaßöffnung 2 des Gehäuses 3 liegt. Der am Umfang des Ventilkörpers 4 verlaufende Kanal 8 trifft hiebei auf die Drosselnut 6 im größten Querschnittsabschnitt 7 derselben.

Ein solcher Durchflußregulator wird in folgender Weise benutzt:

An einen Einlaßstutzen 1 des Gehäuses 3 wird ein Einlaß schlauch montiert und an einen Auslaßstutzen 2 des Gehäuses 3 wird ein Auslaßschlauch angeschlossen.

Wird die Betätigungsscheibe 10 entgegen dem Uhrzeigersinn bis zu einem in der Zeichnung nicht dargestellten Anschlag gedreht, so ist der Auslaß abgesperrt. Wird hingegen die Betätigungsscheibe 10 im Uhrzeigersinn gedreht, so ist ein bestimmter Querschnittsbereich der Drosselnut 6 mit der Auslaßöffnung 2 verbunden, wodurch sich eine bestimmte Anzahl von Tropfen pro Zeiteinheit im Auslaß 2 einstellt. Wird die Betätigungsscheibe 10 weiter bis zum Anschlag aufgedreht, so ist der Durchfluß voll geöffnet. Die verschiedenen Durchflußraten können an einer an der Vorderseite der Betätigungsscheibe 10 angeordneten Skala abgelesen werden.

**Patentansprüche:**

1. Durchflußregulator für Schwerkraftinfusionen bzw.- transfusionen, mit einem Gehäuse (3), das eine Einlaßöffnung (1) und eine am Gehäuseumfang um 180° versetzte Auslaßöffnung (2) für die Flüssigkeit aufweist, und mit einem im Gehäuse (3) angeordneten, relativ zum Gehäuse (3) drehbaren, zylindrischen Ventilkörper (4), dessen Mantel eine Drosselnut (6) mit sich allmählich veränderndem Querschnitt zur Steuerung des Durchflusses zwischen der Einlaßöffnung (1) und der Auslaßöffnung (2) aufweist, welche Drosselnut (6) sich im Ventilkörper (4) in einem achsnormalen Querschnitt über einen teil des Umfanges, insbesondere über einen Winkelbereich von etwa 320°, des Ventilkörpers (4) erstreckt und in einer Ebene liegt, in welcher die Achse der Auslaßöffnung (2) des Gehäuses (3) liegt, so daß stromabwärts betrachtet, je nach Winkelstellung des Ventilkörpers (4) ein größerer oder kleinerer Querschnittsbereich der Drosselnut (6) mit der Auslaßöffnung (2) in Verbindung steht und dadurch eine größere oder Kleinere Anzahl von Tropfen pro Zeiteinheit aus dem Gehäuse (3) abgegeben werden kann, dadurch gekennzeichnet, daß ein vom größten Querschnittsabschnitt (7) der Drosselnut (6) ausgehender, sich insbesondere in Achsrichtung des Ventikörpers (4) erstreckender Kanal (8) in Form einer Mantelnut mit einer sich über 360° erstreckenden Umfangsnut (9) des Ventilköpers (4) in Verbindung steht, in die der Einlaß (1) in das Gehäuse (3) mündet, welcher relativ zum Auslaß (2) in Achsrichtung des Ventilkörpers (4) versetzt ist, so daß, stromaufwärts betrachtet, die Drosselnut (6) über den Kanal (8) und die Umfangsnut (9) mit der Einlaßöffnung (1) in Verbindung steht.

2. Durchflußregulator nach Anspruch 1, dadurch gekennzeichnet, daß der Ventilkörper (4) außerhalb des Gehäuses (3) mit einer Betätigungsscheibe (10) versehen ist.

**Revendications**

1. Régulator de débit pour perfusions ou transfusions par gravité, avec un boîtier (3), présentant un orifice d'admission (1) et un orifice d'échappement du liquide (2) à 180° sur la périphérie du boîtier, et avec une valve cylindrique tournante (4) disposée dans le boîtier (3) tournant par rapport au boîtier (3), valve dont l'enveloppe comporte une rainure d'étranglement (6) dont la section transversale se modifie progressivement pour conduire le débit entre l'orifice d'échappement (2) dont la rainure d'étranglement (6) s'étend dans le corps de valve (4) en une section normale à l'axe, sur une partie de la périphérie, en particulier sur une plage angulaire d'environ 320° du corps de valve (4) une partie plus ou moins grande de la section de la rainure d'étranglement (6) est en communication avec l'orifice d'échappement (2), de sorte que l'on peut débiter un nombre de gouttes plus ou moins grand par unité de temps à partir du boîtier (3), régulateur de débit caractérisé en ce qu'un canal (8) partant d'un tronçon (7) de la section maximale de la rainure d'étranglement (6) s'étendant en particulier dans le sens axial du corps de valve (4), sous forme d'une rainure enveloppe est en communication avec une rainure périphérique (9) s'étendant sur 360°, du corps de valve (4), rainure dans laquelle l'orifice d'admission (1) vient déboucher dans le boîtier (3), l'orifice d'admission étant décalé par rapport à l'orifice d'échappement (2) dans le sens axial du corps de valve (4), de sorte qu'en regardant en amont, la rainure d'étranglement (6) est en communication avec l'orifice d'admission (1) par l'intermédiaire du canal (8) et de la rainure périphérique (9).

2. Régulateur de débit suivant la revendication 1, caractérisé en ce que le corps de valve (4) est muni d'un disque de commande (10) en dehors du boîtier (3).

**Claims**

1. Through-flow regulator for gravity infusions or transfusions, having a housing (3) which has an inlet opening (1) and an outlet opening (2) for the fluid, said outlet opening being offset with respect to the housing periphery by 180°, and having a cylindrical valve body (4) which is arranged in the housing (3), is rotatable relative to the housing (3) and the casing of which has a throttling groove (6) which has gradually changing cross section for the purpose of controlling the through-flow between the inlet opening (1) and the outlet opening (2), extends in the valve body (4) in a transverse cross section over a part of the periphery, more particularly over an angular region of approximately 320° of the valve body (4), and lies in a plane in which the axis of the outlet opening (2) of the housing (3) lies, so that, viewed downstream, depending on the angular position of the valve body (4) a greater or smaller cross sectional region of the throttling groove (6) communicates with the outlet opening (2) and as a result a greater or smaller number of drops can be delivered from the housing (3) per unit of time, characterised in that a channel (8), which originates from the greatest cross sectional portion (7) of the throttling groove (6) and extends more particularly in the axial direction of the valve body (4), shaped as a surface groove communicates with a peripheral groove (9) of the valve body (4), which groove extends over 360° and into which discharges the inlet (1) into the housing (3), which inlet is offset relative to the outlet (2) in the axial direction of the valve body (4) so that, viewed upstream, the throttling groove (6) communicates by way of the channel (8) and the peripheral groove (9) with the inlet opening (1).

2. Through-flow regulator according to claim 1, characterised in that the valve body (4) is provided externally of the housing (3) with an actuating wheel (10).

FIG. 2

FIG. 3

FIG. 4

FIG. 1